# EUROPÄISCHE PATENTSCHRIFT

(11) **EP 1 906 901 B1**
(45) Veröffentlichungstag und Bekanntmachung des Hinweises auf die Patenterteilung: **27.11.2013**
(21) Anmeldenummer: 06754584.8
(22) Anmeldetag: 27.06.2006
(51) Int. Cl.: A61F 13/49, A61F 13/496, A61F 13/494

(54) **WEGWERFBARER ABSORBIERENDER HYGIENEARTIKEL IN PANTFORM**
PANT-TYPE DISPOSABLE, ABSORBENT HYGIENE PRODUCT
ARTICLE HYGIENIQUE ABSORBANT JETABLE EN FORME DE CULOTTE

(30) Priorität: 29.06.2005 DE 102005030182
(43) Veröffentlichungstag der Anmeldung: 09.04.2008
(73) Patentinhaber: Paul Hartmann Aktiengesellschaft, 89522 Heidenheim (DE)
(72) Erfinder: MALOWANIEC, Krzysztof, D., 89522 Heidenheim (DE)
(86) Internationale Anmeldenummer: PCT/EP2006/006192
(87) Internationale Veröffentlichungsnummer: WO 2007/000315

(56) Entgegenhaltungen:
- EP-A- 1 184 017
- EP-A- 1 473 009
- EP-A1- 1 374 814
- EP-A2- 1 163 894
- EP-A2- 1 308 148
- WO-A-01/85082
- WO-A-2004/060238
- WO-A-2004/105668
- US-A1- 2004 260 264

## Beschreibung

Die Erfindung betrifft einen wegwerfbaren absorbierenden Hygieneartikel in Pantform, mit einem eine Hüftöffnung bildenden in Umfangsrichtung durchgehend geschlossenen Hüftrand und mit Beinöffnungen, wobei der in Umfangsrichtung durchgehend geschlossene Hüftrand und die Beinöffnungen gebildet sind, indem Längsseitenrandabschnitte eines Vorderteils und eines Rückenteils herstellerseitig miteinander verbunden sind, und mit einem Absorptionskörper und mit zumindest einem elastischen Beinöffnungsabschnitt und mit Bündchenelementen beidseits des Absorptionskerns, die einen elastischen Bündchenabschnitt aufweisen.

Derartige absorbierende Hygieneartikel in Höschenform sind bekannt. Sie umfassen zumeist eine Vielzahl von Elastifizierungsmitteln, häufig in Form von gummielastischen Fäden, die im vorgespannten Zustand mit Chassismaterialien zumeist klebend verbunden werden. Dabei ist üblicherweise ein Hüftrandbereich vorzugsweise durchgehend in Umfangsrichtung elastifiziert. Auch im Vorderbereich und im Rückenbereich sind bei bekannten Windelhosen Elastifizierungsmittel vorgesehen. Desgleichen werden die Beinöffnungen umgebende bzw. die Beinöffnungen bildende Umfangsbereiche zumindest abschnittsweise elastisch ausgestaltet, damit dort ein weitgehend dichtendes Anliegen des Hygieneartikels an die Hautoberfläche des Benutzers gewährleistet ist, um das seitliche Austreten von Körperausscheidungen zu verhindern. Auch aufstehende Bündchenelemente, die neben den elastischen Beinöffnungen einen weiteren seitlichen Auslaufschutz bieten, werden bei bekannten Windelhosen bereits eingesetzt und sind beispielsweise aus (EP-1184017-A1, EP-1199058-A1, EP-1308148-A2) bekannt.

Wenn unter Vorspannung stehende elastifizierte Bereiche gerade im Bereich der Beinöffnungen oder der Bündchenelemente gegen die Hautoberfläche anliegen, so geht dies oftmals mit Hautreizungen einher, die durch die Reibung oder Krafteinwirkung von zumeist fadenförmigen Elastifizierungsmitteln hervorgerufen werden. Wenn zusätzlich feuchte Bedingungen innerhalb des Hygieneartikels durch Körperflüssigkeiten einerseits oder durch Schweißabsonderungen andererseits hinzukommen, so stellt sich dieses Problem in noch gravierenderem Maße. Es wird weiter durch zunehmende Aktivität, also Bewegungen des Benutzers, verschärft.

Hiervon ausgehend liegt der vorliegenden Erfindung die Aufgabe zugrunde, den Tragekomfort absorbierender Hygieneartikel der eingangs genannten Art zu verbessern und insbesondere das Auftreten von Hautirritationen zu verringern.

Diese Aufgabe wird bei einem gattungsgemäßen Hygieneartikel erfindungsgemäß dadurch gelöst, dass a) ein jeweiliges Bündchenelement zumindest einen nicht elastischen Bündchenabschnitt aufweist und nach dem Anlegen an den Körper des Benutzers der elastische Beinöffnungsabschnitt den jeweiligen nicht elastischen Bündchenabschnitt kreuzt oder b) die Längserstreckung des jeweiligen Bündchenelementes derart bemessen ist, dass nach dem Anlegen an den Körper des Benutzers eine Kreuzung des elastischen Beinöffnungsabschnittes mit dem jeweiligen Bündchenelement unterbleibt.

Da die bevorzugt eingesetzten faden- oder bandförmigen Elastifizierungsmittel eine Raffung oder Rüschung von mit ihnen verbundenen Materialien bewirken, wenn sie im vorgespannten Zustand mit diesen Materialien verbunden werden, führen sie im relaxierten Zustand zu einer Materialanhäufung, die als unangenehm empfunden werden kann. Jedenfalls reiben insbesondere bei Bewegung des Benutzers geraffte oder gerüschte Bereiche, also plastifizierte Bereiche, in besonderem Maße gegen die Hautoberfläche des Benutzers, was zu den vorausgehend erwähnten Hautirritationen führen kann.

Es wurde erfindungsgemäß erkannt, dass eine Vermeidung der Überlappung mehrerer also mindestens zweier elastischer Komponenten in einem Bereich, der unmittelbar an die Haut des Benutzers eng anliegt, die Wahrscheinlichkeit der Bildung von Hautirritationen herabsetzt.

Vor dem Hintergrund, dass moderne und gattungsgemäße Hygieneartikel ohnehin über ein doppeltes seitliches Auslaufschutzsystem verfügen, erscheint es ausreichend, die Bündchenelemente lediglich im zentralen Bereich und über einen kurzen Abschnitt auszubilden oder zumindest nur in diesem zentralen Bereich und über einen kurzen Abschnitt elastisch auszubilden. Hierdurch können somit zudem Materialeinsparungen bei Elastifizierungsmitteln und Fügematerialien wie Klebstoffen realisiert werden.

In einer ersten Variante der Erfindung werden die Bündchenelemente derart kurz und lediglich in einem zentralen Schrittbereich angeordnet, dass nach dem Anlegen des Artikels an den Benutzer ein sich Kreuzen, das heißt ein abschnittsweises Überlappen von Bündchenelement und elastischem Beinöffnungselement vermieden wird.

In einer zweiten Variante der Erfindung umfasst ein jeweiliges Bündchen einen elastischen und einen nicht elastischen Bündchenabschnitt und die Anordnung dieser Abschnitte ist derart, dass die elastischen Beinöffnungsabschnitte die Bündchenelemente in deren unelastischem Abschnitt kreuzen.

Da Windelhosen üblicherweise sowohl im vorderen als auch im hinteren Beinöffnungsbereich elastische Beinöffnungsabschnitte aufweisen, sieht eine Weiterbildung der Erfindung vor, dass das jeweilige Bündchenelement mindestens zwei nicht elastifizierte Abschnitte aufweist. Diese sind den jeweiligen Hüfträndem näher als der elastische Bündchenabschnitt, und dort angeordnet, wo elastischer Beinöffnungsabschnitt und Bündchenelement nach dem Anlegen des Artikels an den Benutzer überlappen, das heißt sich kreuzen.

Eine Weiterbildung der Erfindung sieht vor, ein jeweiliges Bündchenelement mit mehreren, also mindestens zwei elastischen Bündchenabschnitten zu versehen. Solchenfalls sind diese elastischen Bündchenabschnitte durch nicht elastische Bündchenabschnitte voneinander beabstandet. Dies hat den Vorteil der höheren Flexibilität in der Anordnung und Bemessung der Länge der Bündchenelemente. Sollten beispielsweise sehr lange Absorptionskörper verwendet werden, kann ein sicheres Aufstehen der Bündchenelemente nur durch einen entsprechend langen Elastifizierungsabschnitt sichergestellt werden. Solchenfalls können die nicht elastifizierten Bündchenabschnitte Bereiche zwischen zwei elastifizierten Bündchenabschnitten bilden, in denen die elastischen Beinöffnungsabschnitte die Bündchenelemente nach dem Anlegen des Artikels an den Benutzer kreuzen können.

Die elastischen Bündchenabschnitte und/oder die elastischen Beinöffnungsabschnitte werden in bekannter Weise vorzugsweise durch Vlies- oder Folienmaterialien gebildet, welche durch insbesondere faden- oder bandförmige Elastifizierungsmittel, wie Gummi- oder Polyetherpolyurethan- oder Polyesterpolyurethanfäden elastifiziert sind. Hierbei werden die Elastifizierungsmittel vorzugsweise in vorgespanntem Zustand auf den Vlies- oder Folienmaterialien fixiert (Stretch-Bonding), so dass über die Rückstellkraft der Elastifizierungsmittel nach Lösen der Vorspannung eine elastisch dehnbare Raffung der entsprechenden Abschnitte erzielt wird.

Der oder die nicht elastischen Bündchenabschnitte werden insbesondere dadurch erhalten, dass dort keine Elastifizierungsmittel vorgesehen werden. Dies spart Materialkosten und vermeidet am sichersten die zu Hautirritationen führende bereits erläuterte Überlappungsproblematik nach Anlegen der Windel.

Der oder die nicht elastischen Bündchenabschnitte können aber auch insbesondere dadurch erhalten werden, dass die Elastifizierungsmittel in diesen Abschnitten entspannt verlaufen.

Der oder die nicht elastischen Bündchenabschnitte können insbesondere dadurch erhalten werden, dass die Elastifizierungsmittel in diesen Abschnitten nicht mit den Bündchenelementen verbunden sind, so dass beim zuvor erläuterten "Stretch-bonding" und nachfolgendem Lösen der Spannung die Elastifizierungsmittel in diesen Abschnitten ohne Spannung angeordnet sind und somit keine Kraft auf die Bündchenelemente entfalten und somit in diesen Abschnitten keine Raffung der Bündchenelemente erfolgt. Zur lediglich abschnittsweisen Elastifizierung von Flachmaterialkomponenten durch faden- oder bandförmige Elastifizierungsmittel wird ergänzend auf den Offenbarungsgehalt der DE-2649948-A1 verwiesen.

Der erfindungsgemäße Hygieneartikel weist vorzugsweise eine das Windelchassis bildende äußere Hülle auf, die einen Vorderteil, einen Rückenteil und einen dazwischen angeordneten mit den Beinöffnungen versehenen Schrittteil umfasst. Die äußere Hülle wird vorzugsweise aus Vliesstoff- und/oder Folienmaterialien gebildet. Um eine Luft- und/oder Wasserdampfzirkulation zu gewährleisten werden vorzugsweise ausschließlich Vliesstoffmaterialien eingesetzt. Die äußere Hülle kann insbesondere als ein kontinuierlicher sich von einem vorderen Hüftrand zu einem hinteren Hüftrand erstreckender Materialabschnitt ausgebildet sein. Denkbar und vorteilhaft ist aber auch, wie dies beispielsweise bereits WO-2004-060238 lehrt, die äußere Hülle aus einem vorderen Hüllenteil und einem hinteren Hüllenteil zu bilden. Solchenfalls sind vorderer Hüllenteil und hinterer Hüllenteil im Schrittbereich voneinander beabstandet. Der Absorptionskern ist in dieser Ausführungsform mit einem Vorderbereich am vorderen Hüllenteil und mit einem hinteren Bereich an dem hinteren Hüllenteil verbunden, derart das vorderer Hüllenteil und hinterer Hüllenteil lediglich indirekt über den Absorptionskern miteinander chassisbildend verbunden sind.
In Weiterbildung dieser Ausführungsform kann der Absorptionskern mit der körperfernen Seite des jeweiligen vorderen oder hinteren Bereiches auf der körpernahen Seite des jeweiligen Hüllenteils verbunden sein. In einer alternativen Weiterbildung kann die Anordnung umgekehrt sein, das heißt es kann der Absorptionskern mit der körpernahen Seite des vorderen oder hinteren Bereiches auf der körperfernen Seite des jeweiligen Hüllenteils verbunden sein. Schließlich kann der Absorptionskern mit seinen vorderen und/oder hinteren Bereichen auch sandwichartig zwischen den körperfernen und körpernahen Schichten des solchenfalls mehrschichtigen vorderen und/oder hinteren Hüllenteils angeordnet und verbunden sein.

Im Falle einer kontinuierlichen äußeren Hülle kann der Absorptionskern als integraler Bestandteil der äußeren Hülle, insbesondere zwischen einer körpernahen Hüllenschicht und einer körperfernen Hüllenschicht angeordnet sein.

In einer bevorzugten Ausführungsform ist der Absorptionskörper mit seiner körperfernen Seite auf der körpernahen Schicht der äußeren Hülle angeordnet und vorzugsweise daran durch Klebemittel, wie Heißschmelzklebemittel oder andere Fügeverfahren, wie thermisches Schweißen zumindest abschnittsweise fixiert.

Der Absorptionskern umfasst zumindest einen Speicherkern mit Körperflüssigkeiten absorbierenden Materialien wie natürlichen oder synthetischen Fasern, insbesondere Cellulosefasern, vorzugsweise in Form von Zellstofffluff. Vorzugsweise umfasst der Speicherkern außerdem superabsorbierende Materialien (SAP), insbesondere auf Basis oberflächenvernetzter, teilneutralisierter Polyacrylate. Der Absorptionskörper wird vorzugsweise von einem zumindest abschnittsweise flüssigkeitsdurchlässigen Topsheet überfangen und weiter vorzugsweise von einem zumindest abschnittsweise in Gebrauch flüssigkeitsundurchlässigem Backsheet unterfangen. Das Topsheet umfasst insbesondere Vliesstoffmaterialien oder mit Öffnungen versehene Folienmaterialien. Das Backsheet umfasst insbesondere eine Folie, insbesondere einer Dicke von höchstens 15 µm. Das Backsheet kann vorteilhafterweise aber auch in Gebrauch flüssigkeitsdichte gleichwohl aber wasserdampfdurchlässige Vliesstoffmaterialien wie Meltblownschichten (M) und Spunbondschichten (S), insbesondere Laminate aus Meltblown- und Spunbondschichten, wie SM- oder SMS- oder SMMS-Laminate, enthalten oder daraus bestehen. Insbesondere umfasst das Backsheet eine in Gebrauch flüssigkeitsdichte aber gleichwohl atmungsaktive, also wasserdampfdurchlässige, insbesondere mikroporöse Folie. Die Wasserdampfdurchlässigkeit des Backsheets beträgt insbesondere wenigstens 300g/m²/24h , weiter insbesondere wenigstens 1000g/m²/24h, weiter insbesondere wenigstens 2000g/m²/24h, weiter insbesondere wenigstens 3000g/m²/24h, weiter insbesondere wenigstens 4000g/m²/24h, weiter insbesondere höchstens 6000g/m²/24h gemessen nach DIN 53 122-1 (Ausgabe: 2001-08).

Die Bündchenelemente erstrecken sich beidseits des Absorptionskerns und sind insbesondere mit ihren proximalen Rändern an einem jeweiligen Längsrand der körpernahen Seite des Absorptionskerns, insbesondere dem Topsheet festgelegt. In einer alternativen Weiterbildung der Erfindung sind die proximalen Ränder der Bündchenelemente beidseits außerhalb des Absorptionskerns, das heißt auf der körpernahen Seite der äußeren Hülle festgelegt.
Die distalen, zum Benutzer hin zumindest abschnittsweise aufstehenden Ränder weisen vorzugsweise Elastifizierungsmittel, insbesondere faden- oder bandförmige Elastifizierungsmittel der vorgenannten Art auf. In einer alternativen Ausführungsform umfassen oder bestehen die Bündchenelemente aus massiv elastischem Material, wie einer elastischen Folie oder einem elastischen Vliesstoff.
Vorzugsweise ist der distale Rand der Bündchenelemente nach innen in Richtung auf die Längsachse des Hygieneartikels geneigt. Dies wird insbesondere dadurch sichergestellt, dass der distale Rand der Bündchenelemente in einem oder beiden Endabschnitten nach innen auf der körperzugewandte Seite des Absorptionskerns, insbesondere auf das Topsheet festgelegt ist. Durch die Festlegung der Endabschnitte der Bündchenelemente über einen genügend großen Abschnitt auf dem Absorptionskern kann in einer bevorzugten Ausführungsform diesem Abschnitt gleichzeitig die elastische Wirkung genommen werden, womit dieser festgelegte Endabschnitt als koinzidierend mit einem nicht elastifizierten Bündchenabschnitt anzusehen ist.

Die Beinöffnungen eines erfindungsgemäßen Hygieneartikels weisen mindestens einen elastischen Beinöffnungsabschnitt auf. Vorzugsweise verläuft ein elastischer Beinöffnungsabschnitt einer jeweiligen Beinöffnung - im flachgelegten Zustand vor der herstellerseitigen Verbindung der Längsseitenrandabschnitte betrachtet - ausgehend von einem Längsseitenrand des Vorderteils oder Rückenteils des Artikels in einer Kurve entlang eines Umfangs einer jeweiligen Beinöffnung in Richtung auf eine Schrittmittellinie. Die gekrümmte Anordnung des elastischen Beinöffnungsabschnitts unterstützt die körpergerechte Anpassung des Hygieneartikels in besonderer Weise. Vorzugsweise ist an den ersten Beinöffnungsabschnitt anschließend ein zweiter elastischer Beinöffnungsabschnitt vorgesehen.

Der zweite Beinöffnungsabschnitt ist vorzugsweise durch faden- oder bandförmige Elastifizierungsmittel der zuvor genannten Art elastifiziert und verläuft insbesondere im Wesentlichen parallel zur zentralen Längsachse (L). Dies ermöglicht, den zweiten Beinöffnungsabschnitt durch beidseits an den Absorptionskern gebundene Elastifizierungsmittel zu bilden. In einer bevorzugten Ausführungsform, kann der zweite elastische Beinöffnungsabschnitt somit durch die elastischen Längsränder des Absorptionskerns plastifiziert sein. Alternativ kann der zweite elastische Beinöffnungsabschnitt durch beidseits außerhalb des Absorptionskerns angeordnete, also direkt mit der äußeren Hülle verbundene Elastifizierungsmittel elastifiziert sein.

Vorzugsweise verläuft ein weiterer, dritter elastischer Beinöffnungsabschnitt einer jeweiligen Beinöffnung - wiederum im flachgelegten Zustand vor der herstellerseitigen Verbindung der Längsseitenrandabschnitte betrachtet - ausgehend von einem Längsseitenrand des Vorderteils oder Rückenteils des Artikels in einer Kurve entlang eines Umfangs einer jeweiligen Beinöffnung in Richtung auf eine Schrittmittellinie.

In einer bevorzugten Ausführungsform weist eine jeweilige Beinöffnung die zuvor beschriebenen ersten, zweiten und dritten eiastifizierten Beinöffnungsabschnitte auf, derart, dass die ersten und dritten Beinöffnungsabschnitte in Längsrichtung des Hygieneartikels einen erheblichen Abstand aufweisen, insbesondere einen Abstand von mindestens 10 cm, insbesondere von mindestens 15 cm, weiter insbesondere von mindestens 18 cm, weiter insbesondere von höchstens 30 cm. Die zweiten elastischen Beinöffnungsabschnitte sind derart angeordnet, dass sie diesen Abstand zumindest abschnittsweise überbrücken. Um eine gegenseitige Störung der elastischen Wirkungen der elastischen Beinöffnungsabschnitte und eine mit zu Hautirritationen führende Materialanhäufung zu vermeiden, sind die Endbereiche des jeweiligen zweiten elastischen Beinöffnungsabschnitts von den Endbereichen des jeweiligen ersten und/oder dritten elastischen Beinöffnungsabschnitts vorzugsweise beabstandet, und zwar vorzugsweise um eine Länge A, wobei die Länge A vorzugsweise 10 mm, insbesondere mindestens 15 mm, weiter insbesondere mindestens 18 mm beträgt.

Um erfindungsgemäß ein Überlappen bzw. sich Kreuzen der elastischen Beinöffnungsabschnitte mit den elastischen Bündchenabschnitten zu vermeiden, hat sich herausgestellt, dass insbesondere eine Anordnung und Dimensionierung der genannten Abschnitte vorteilhaft ist, die dadurch gekennzeichnet ist, dass die Längserstreckung des elastischen Bündchenabschnittes um höchstens 8 cm, insbesondere um höchstens 6 cm, insbesondere um höchstens 4 cm, insbesondere um höchsten 2 cm größer ist als die Längserstreckung des Abstandes C zwischen dem ersten elastischen Beinöffnungsabschnitt und dem dritten elastischen Beinöffnungsabschnitt. Besonders vorteilhaft ist eine Anordnung bei der die Längserstreckung des elastischen Bündchenabschnittes gleich oder kürzer, insbesondere um mindestens 1 cm kürzer, weiter insbesondere um mindestens 2cm kürzer, weiter insbesondere um höchstens 4 cm kürzer ist als die Längserstreckung des Abstandes C zwischen dem ersten elastischen Beinöffnungsabschnitt und dem dritten elastischen Beinöffnungsabschnitt.

Es erweist sich ferner als vorteilhaft, wenn zwischen Hüftrand und Schrittbereich im Wesentlichen in einer Querrichtung erstreckte Elastifizierungsmittel vorgesehen sind, um die Passform des Hygieneartikels im Sinne einer körpernahen Anordnung zu verbessern. Ein solches Elastifizierungsmittel kann im Bereich des Absorptionskörpers zu einer gewollten Zusammenziehung des Saugkörpers im Schrittbereich führen.

Es erweist sich ferner ebenfalls als vorteilhaft, wenn im Schrittbereich im Wesentlichen in einer Querrichtung erstreckte Elastifizierungsmittel vorgesehen sind. Es kann nämlich durchaus gewünscht sein, dass der Absorptionskörper im Schrittbereich schmal ausgebildet ist, aber dennoch eine hinreichende Absorptionskapazität, also absorbierendes Material, zur Verfügung stellt. Durch eine Zusammenziehung kann dann das Flächengewicht in diesem Bereich erhöht werden, um in diesem Bereich eine große Absorptionskapazität zur Verfügung zu stellen. Außerdem führen im Schrittbereich in Querrichtung verlaufende Elastifizierungsmittel zu einer Formgebung des Hygieneartikels. Es wird in vorteilhafter Weise eine Schalenform im Schrittbereich erzeugt.

Das im Schrittbereich im Wesentlichen in Querrichtung verlaufende Elastifizierungsmittel oder die Elastifizierungsmittel könnten separat von denjenigen Elastifizierungsmitteln, welche die Beinöffnungen elastifizieren, gebildet sein. Nach einer weiteren Ausführungsform der Erfindung erweist es sich als vorteilhaft, dass die Elastifizierungsmittel des elastifzierten Beinöffnungsabschnittes, insbesondere des ersten und/oder dritten elastifizierten Beinöffnungsabschnittes in die Elastifizierungsmittel des Schrittbereichs übergehen, d. h. das oder die in Querrichtung verlaufenden Elastifizierungsmittel im Schrittbereich bilden, indem sie ausgehend von ihrer Erstreckung in Umfangsrichtung der Beinöffnungen dann über einen Wendepunkt im Wesentlichen in die Querrichtung übergehen. Sie können dabei in einem im Wesentlichen stetigen Kurvenverlauf oder auch unstetig abzweigen, wobei einem stetigen Verlauf der Vorzug zu geben ist.

Falls - zum Beispiel aus optischen Gründen - keine Raffung des Absorptionskerns im Schrittbereich gewünscht ist, können diese Elastifizierungsmittel, insbesondere in einem Bereich nach Durchlauf des Wendepunktes ihrer elastifizierenden Wirkung benommen insbesondere durchtrennt werden. Die Elastifizierungsmittel werden dann relaxieren bzw. zurückschnellen, soweit sie in diesem Abschnitt keine Verbindung zur äußeren Hülle aufweisen.
Die Durchtrennung kann beispielsweise durch einen einzigen Schnitt erfolgen. Es wäre aber auch denkbar und vorteilhaft, dass die Durchtrennung der Elastifizierungsmittel durch eine Vielzahl von Schnitten erfolgt, so dass die zuvor durchgehend erstreckten Elastifizierungsmittel in eine Vielzahl von kleinen Abschnitte, insbesondere einer Länge im Millimeterbereich, geteilt werden und so ihre elastifizierende Wirkung verlieren. Solchenfalls können die Elastifizierungsmittel auch klebend mit der äußeren Hülle verbunden sein, ohne dass dies die Relaxierung behindern würde. Ein einfaches oder mehrfaches Durchtrennen der Elastifizierungsmittel kann auch mittels Lasertechnik erfolgen. In einer alternativen Ausführungsform werden die zuvor erstreckten Elastifizierungsmittel durch Anwendung von Hitze und/oder Druck und/oder Ultraschall ihrer elastischen Wirkung benommen.
Die Anwendung von Hitze und/oder Druck bringt gegenüber der Anbringung von Trennschnitten den Vorteil mit sich, dass hierbei weniger die Gefahr besteht, dass die äußere Hülle bildende Materialien in ungewollter Weise mitgeschnitten oder perforiert werden.

Zur Offenbarung einer Technik der Anwendung von Hitze und/oder Druck zur Inaktivierung von Elastifizierungsmitteln wird auf EP 1 374 814 A1 verwiesen, deren Inhalt insoweit zum Gegenstand der vorliegenden Anmeldung gemacht wird.

Weitere Merkmale, Einzelheiten und Vorteile der Erfindung ergeben sich aus den beigefügten Patentansprüchen und aus der zeichnerischen Darstellung und nachfolgenden Beschreibung bevorzugter Ausführungsformen des erfindungsgemäßen Hygieneartikels.

In der Zeichnung zeigt:
- Figur 1: eine Draufsicht auf eine erste Ausführungsform eines erfindungsgemäßen Hygieneartikels im flachgelegten Zustand vor der herstellerseitigen Verbindung von Längsseitenrandbereichen;
- Figur 2: eine erste Schnittansicht des in Figur 1 dargestellten Hygieneartikels entlang einer Ebene F-F
- Figur 3: eine zweite Schnittansicht des in Figur 1 dargestellten Hygieneartikels entlang einer Ebene G-G
- Figur 4: eine perspektivische Darstellung einer erfindungsgemäßen Windel im an den Benutzer angelegten Zustand

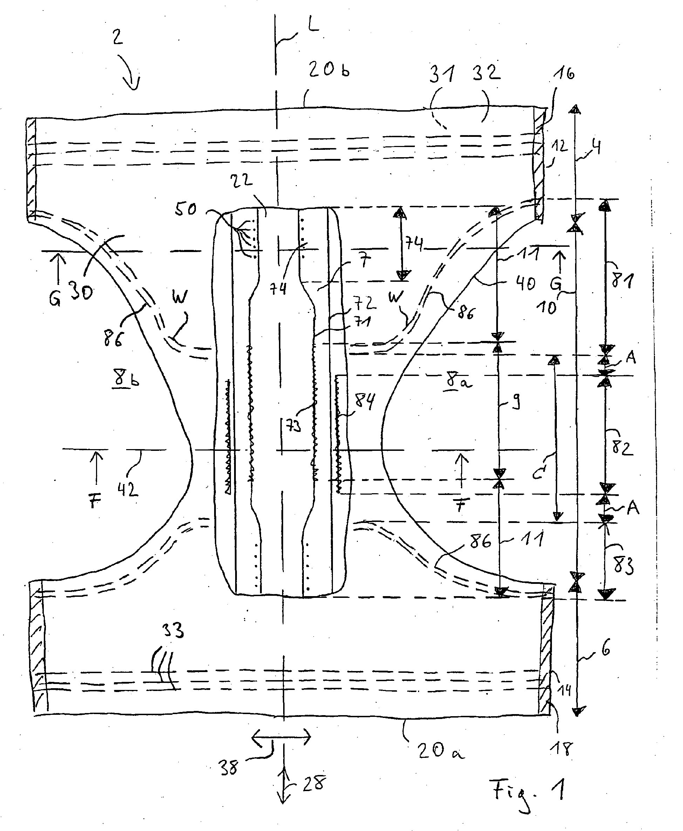

Figur 1 zeigt einen insgesamt mit dem Bezugszeichen 2 bezeichneten Hygieneartikel in Form einer Windelhose ("pant"), jedoch im flachgelegten Zustand vor der herstellerseitigen Verbindung der Längsseitenrandabschnitte. Der Hygieneartikel umfasst einen Rückenteil 4, einen Vorderteil 6 und einen dazwischen liegenden, die späteren Beinausschnitte 8 a, b begrenzenden Schrittteil 10. Im flachgelegten Zustand wird der Rückenteil 4 von Längsseitenrändern 12 und der Vorderteil 6 von Längsseitenrändern 14 begrenzt. Diese werden zur Bildung der Pant-Form im Bereich der schraffierten Zonen 16 bzw. 18 miteinander verbunden, etwa durch Heißsiegeln oder sonstige übliche Fügeverfahren. Auf diese Weise werden zwei Seitennahtbereiche des Hygieneartikels gebildet, die nicht zerstörungsfrei gelöst werden können. Der fertig konfigurierte Hygieneartikel kann aber durchaus, insbesondere entlang dieser Seitennahtbereiche, eine Sollbruchlinie, beispielsweise in Form eines Reißfadens aufweisen, um den Hygieneartikel ausgehend vom angelegten Zustand öffnen zu können. Auch können zusätzlich an sich beliebige Verschlusselemente, etwa klebende oder mechanisch haftende Verschlusslaschen, vorgesehen sein, um den Hygieneartikel nach dem Öffnen wieder wie eine Windel schließen zu können. Herstellerseitig ist der Hygieneartikel aber als Höschen ("pant") gefertigt und weist daher einen in Umfangsrichtung geschlossenen Hüftrand 20 auf, der eine allseits begrenzte Hüftöffnung bildet.

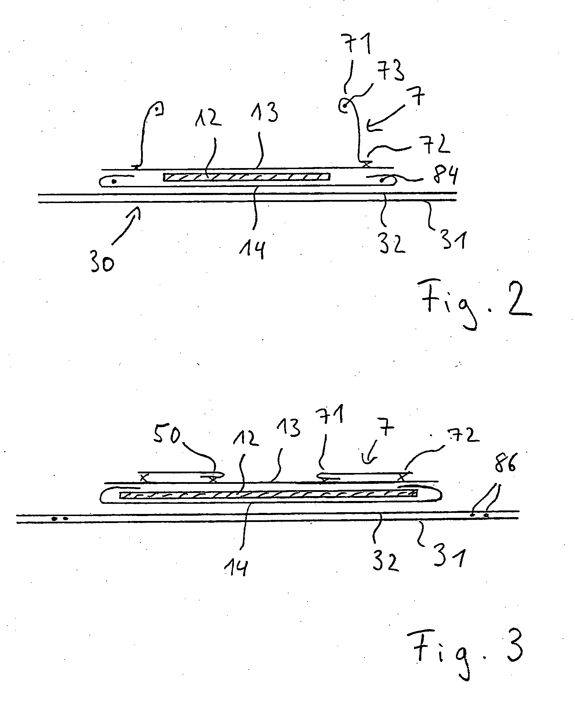

Der Hygieneartikel 2 umfasst des Weiteren einen Absorptionskörper 22, der als funktionelle und vorgefertigte Einheit ausgebildet ist, d. h. der Absorptionskern weist einen flüssigkeitsabsorbierenden und -speichernden Speicherkern 12, ein flüssigkeitsundurchlässige Backsheet 14 und ein flüssigkeitsdurchlässiges Topsheet 13 auf. Die Bündchenelemente 7 sind aus einem hydrophoben Vliesmaterial gebildet und sind mit ihrem proximalen Rand 72 beidseits des Speicherkerns 12 parallel zur Längsachse L auf dem Topsheet 13 festgelegt. Der jeweilige distale Rand 71 der Bündchenelemente 7 ist in einem das Schrittzentrum umfassenden Abschnitt, dem elastischen Bündchenabschnitt 9, mit einem Elastifizierungsmittel 73, nämlich einem elastischen Gummifaden versehen. Vermittels der Rückstellkraft des Gummifadens kann der elastische Bündchenabschnitt 9 wie Figur 2 zeigt nach oben gegen die Haut des Benutzers aufstehen. In Richtung des vorderen und hinteren Hüftrandes 20a, 20b verbleibt das jeweilige Bündchenelement unelastifiziert und bildet dort somit nicht elastifizierte Abschnitte 11. Der distale Rand 71 ist an einem hinteren und vorderen Endabschnitte 74 des Bündchenelementes 7 nach innen umgelegt und auf dem Topsheet durch die Ultraschallschweißpunkte 50 festgelegt. Der Absorptionskern umfasst außerdem parallel zur Längsachse L orientierte Elastifizierungsmittel 84 die, wie Figur 2 zeigt, in dem umgefalteten Längsrand des Backsheets durch Schmelzklebstoff fixiert sind. Denkbar wäre auch, die Anordnung der Elastifizierungsmittel 84 direkt zwischen Topsheet 13 und Backsheet 14 vorzusehen. Vorzugsweise sind die Elastifizierungsmittel 84 in Querrichtung 38 betrachtet außerhalb des proximalen Randes 72 der Bündchenelemente 7 angeordnet, das heißt die Elastifizierungsmittel 84 sind näher an den Beinöffnungen 8 a, b angeordnet als es die proximalen Ränder 72 der Bündchenelemente 7 sind.

Der Absorptionskern ist als funktionale und vorgefertigte Einheit mit seiner körperfernen Seite, also mit der körperabgewandten Seite des Backsheets 14 auf der körpernahen Seite der äußeren Hülle verbunden. Die das Windelchassis bildende äußere Hülle 30 erstreckt sich kontinuierlich vom vorderen Hüftrand 20a zum hinteren Hüftrand 20b und umfasst vorliegend ein zweilagiges Vliesstofflaminat bestehend aus einer körpernahen Vliesstoffschicht 32 und einer körperfernen Vliesstoffschicht 31, die zumindest abschnittsweise verbunden sind. Zwischen einem jeweiligen Hüftrand 20a, 20b und dem Beginn der Beinöffnungen 8 a, b erstrecken sich in Querrichtung 38 elastische Gummifäden 33, die zwischen den beiden Vliesstoffschichten 31, 32 vorgespannt durch Schmelzhaftklebstoff fixiert sind. Die Hüftränder 20a, 20b weisen ebenfalls - hier nicht dargestellte - sich in Querrichtung 38 erstreckende Elastifizierungsmittel auf.

Man erkennt des Weiteren Elastifizierungsmittel 86, welche die Beinöffnungen 8 a, b abschnittsweise elastifizieren. Auch diese als Gummifäden ausgeführten Elastifizierungsmittel 86 sind zwischen den beiden Vliesstoffschichten 31, 32 vorgespannt durch Schmelzhaftklebstoff fixiert. Die Elastifizierungsmittel 86 erstrecken sich ausgehend vom Längsseitenrand 12 des Rückenteils 4 in einer Kurve entlang eines Umfangs 40 der betrachteten Beinöffnung 8 a, b in Richtung auf eine Schrittmittellinie 42. Die elastifizierende Wirkung der Elastifizierungsmittel 86 ist aber nicht durchgehend bis zur Schrittmittellinie 42, sondern sie ist ausgehend vom Längsseitenrand 12 auf einen ersten Beinöffnungsabschnitt 81 beschränkt. Die Elastifizierungsmittel 86 gehen nämlich ausgehend von ihrer Erstreckung in Umfangsrichtung der Beinöffnungen 8 a über einen Wendepunkt W im Wesentlichen in die Querrichtung 38 über. Sie traversieren nachfolgend den Schrittbereich unterhalb des Absorptionskerns 22, um auf der gegenüberliegenden Seite einen spiegelbildlichen Verlauf entlang der weiteren zweiten Beinöffnung 8b zu nehmen. Unterhalb des Absorptionskerns sind die Elastifizierungsmittel 86 jedoch durch Anwendung von Druck und Hitze ihrer elastischen Wirkung benommen worden.

Ausgehend vom Längsseitenrand 14 des Vorderteils 6 entlang eines Umfangs 40 der betrachteten Beinöffnung 8 a, b in Richtung auf eine Schrittmittellinie 42 erstrecken sich ebenfalls Elastifizierungsmittel 86. Auch die elastifizierende Wirkung der Elastifizierungsmittel 86 im Vorderteil 6 ist aber nicht durchgehend bis zur Schrittmittellinie 42, sondern sie ist ausgehend vom Längsseitenrand 12 auf einen dritten Beinöffnungsabschnitt 83 beschränkt. Wiederum gehen nämlich die Elastifizierungsmittel 86 ausgehend von ihrer Erstreckung in Umfangsrichtung der Beinöffnung 8a über einen Wendepunkt W noch vor Erreichen der Schrittmittellinie 42 im Wesentlichen in die Querrichtung 38 über. Sie traversieren nachfolgend den Schrittbereich unterhalb des Absorptionskerns 22, um auf der gegenüberliegenden Seite einen spiegelbildlichen Verlauf entlang der weiteren zweiten Beinöffnung 8b zu nehmen. Unterhalb des Absorptionskerns sind die Elastifizierungsmittel 86 jedoch durch Anwendung von Druck und Hitze ihrer elastischen Wirkung benommen worden.

Erster 81 und dritter Beinöffnungsabschnitt 83 weisen somit einen sich in Längsrichtung 28 erstreckenden Abstand C auf, der vorliegend 23 cm beträgt.

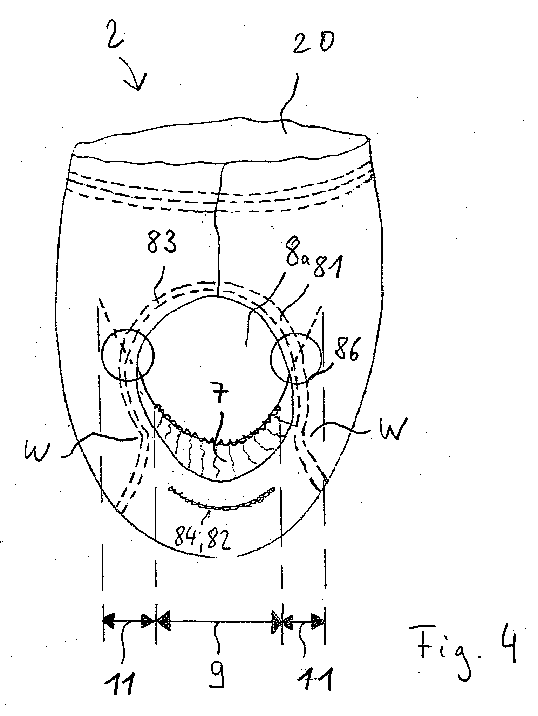

Man erkennt außerdem die zuvor bereits beschriebenen Elastifizierungsmittel 84, die den zweiten elastifizierten Beinöffnungsabschnitt 82 bilden und die diesen Abstand C teilweise ausfüllen. Zwischen den Endbereichen des zweiten elastischen Beinöffnungsabschnittes 82 und Endbereichen des ersten 81 und dritten elastischen Beinöffnungsabschnittes 83 verbleibt jedoch ein Abstand A von 20 mm. Hierdurch lässt sich eine gegenseitige Störung der elastischen Wirkungen vermeiden. Auch wird hierdurch eine übereinander angeordnete Anhäufung elastischen Materials, die ebenfalls zu Hautirritationen führen kann, vermieden. Figur 4 zeigt in einer perspektivischen Ansicht auf die Beinöffnung 8a die Anordnung der mit Elastifizierungsmitteln 86 versehenen ersten und dritten elastischen Beinöffnungsabschnitte 81, 83 und dem elastischen Bündchenabschnitt 9 nach dem Anlegen des in Figuren 1-3 dargestellten Hygieneartikels 2 an den Benutzer, wobei der Benutzer selbst nicht dargestellt ist. Um einen Teil der Beinöffnung 8a herum sind die ersten 81, zweiten 82 und dritten 83 elastischen Beinöffnungsabschnitte erkennbar. Erkennbar sind außerdem die Bündchenelemente 7 mit dem elastischen Bündchenabschnitt 9 sowie den nicht elastischen Bündchenabschnitten 11. Eine Kreuzung der elastischen Beinöffnungsabschnitte mit dem elastischen Bündchenabschnitt wird sicher vermieden. Die Kreuzung der ersten 81 und zweiten 83 elastischen Beinöffnungsabschnitte mit den Bündchenelementen 7 erfolgt lediglich mit deren nicht elastischen Bündchenabschnitten 11 innerhalb der in Figur 4 angedeuteten gedachten Kreise. Hierdurch wird eine Materialanhäufung gerafften Materials in einem an das Bein des Benutzers eng und unter Spannung anliegenden Bereich und damit die Gefahr der Hautirritation reduziert.

## Patentansprüche

1. Wegwerfbarer absorbierender Hygieneartikel (2) in Panttorm, mit einem eine Hüftöffnung bildenden in Umfangsrichtung durchgehend geschlossenen Hüftrand (20) und mit Beinöffnungen (8 a, b), und mit Längsseitenrandabschnitte (16, 18) aufweisendem Vorderteil (6) und Rückenteil (4), und mit einem zwischen Vorderteil (6) und Rückenteil (4) angeordneten Schrittbereich (10), wobei der in Umfangsrichtung durchgehend geschlossene Hüftrand (20) und die Beinöffnungen (8 a, b) gebildet sind, indem Längsseitenrandabschnitte (16, 18) eines Vorderteils (6) und eines Rückenteils (4) herstellerseitig miteinander verbunden sind, und mit einem Absorptionskörper (22), wobei eine jeweilige Beinöffnung (8 a, b) einen elastischen Beinöffnungsabschnitt (81) aufweist und wobei beidseits des Absorptionskörpers (22) und in dessen Längsrichtung (28) erstreckt Bündchenelemente (7) vorgesehen sind, wobei ein jeweiliges Bündchenelement (7) einen elastischen Bündchenabschnitt (9) aufweist und wobei
a) ein jeweiliges Bündchenelement (7) zumindest einen nicht elastischen Bündchenabschnitt (11) aufweist und nach dem Anlegen an den Körper des Benutzers der elastische Beinöffnungsabschnitt (81) den jeweiligen nicht elastischen Bündchenabschnitt (11) kreuzt oder
b) die Längserstreckung des jeweiligen Bündchenelementes (7) derart bemessen ist, dass nach dem Anlegen an den Körper des Benutzers eine Kreuzung des elastischen Beinöffnungsabschnittes (81) mit dem jeweiligen Bündchenelement (7) unterbleibt.

2. Hygieneartikel nach Anspruch 1 **dadurch gekennzeichnet, dass** ein jeweiliges Bündchenelement (7) zwei nicht elastische Bündchenabschnitte (11) aufweist, wobei ein erster nicht elastischer Bündchenabschnitt näher zum vorderen Hüftrand (20a) und ein zweiter nicht elastischer Bündchenabschnitt näher zum hinteren Hüftrand (20b) angeordnet ist als der elastische Bündchenabschnitt (9).

3. Hygieneartikel nach Anspruch 1 oder 2, **dadurch gekennzeichnet, dass** ein jeweiliges Bündchenelement (7) einen einzigen, zumindest abschnittsweise im Schrittbereich (10) positionierten elastischen Bündchenabschnitt (9) aufweist.

4. Hygieneartikel nach einem der vorstehenden Ansprüche **dadurch gekennzeichnet, dass** ein jeweiliges Bündchenelement (7) zumindest zwei elastische Bündchenabschnitte aufweist, wobei die elastischen Bündchenabschnitte durch nicht elastische Bündchenabschnitte voneinander beabstandet sind.

5. Hygieneartikel nach einem der vorstehenden Ansprüche, **dadurch gekennzeichnet, dass** der elastische Bündchenabschnitt (9) und/oder der elastische Beinöffnungsabschnitt (81) durch faden- oder bandförmigen Elastifizierungsmittel (73, 86) elastifiziert ist oder sind.

6. Hygieneartikel nach einem der vorstehenden Ansprüche, **dadurch gekennzeichnet, dass** der nicht elastische Bündchenabschnitt (11) dadurch erhalten ist, dass dort zuvor erstreckte Elastifizierungsmittel durchtrennt worden sind.

7. Hygieneartikel nach Anspruch 6, **dadurch gekennzeichnet, dass** die Durchtrennung durch eine Vielzahl von Schnitten erzielt ist.

8. Hygieneartikel nach einem oder mehreren der vorstehenden Ansprüche, **dadurch gekennzeichnet, dass** der der nicht elastische Bündchenabschnitt (11) dadurch erhalten ist, dass dort zuvor erstreckte Elastifizierungsmittel durch Anwendung von Hitze und/oder Druck und/oder Lasereinwirkung und/oder Ultraschall ihrer elastifizierenden Wirkung benommen worden sind.

9. Hygieneartikel nach einem der vorstehenden Ansprüche, **dadurch gekennzeichnet, dass** der nicht elastische Bündchenabschnitt (11) dadurch erhalten ist, dass dort sich erstreckende Elastifizierungsmittel ohne Spannung angeordnet sind.

10. Hygieneartikel nach einem der vorstehenden Ansprüche, **dadurch gekennzeichnet, dass** der nicht elastische Bündchenabschnitt (11) dadurch erhalten ist, dass dort zumindest abschnittsweise keine Elastifizierungsmittel angeordnet sind.

11. Hygieneartikel nach einem der vorstehenden Ansprüche, **dadurch gekennzeichnet, dass** die Bündchenelemente (7) einen distalen (71) und einen proximalen Rand (72) aufweisen und der elastische Bündchenabschnitt durch die am distalen Rand angeordneten faden- oder bandförmigen Elastifizierungsmittel (73) elastifiziert ist.

12. Hygieneartikel nach einem der vorstehenden Ansprüche, **dadurch gekennzeichnet, dass** der Absorptionskörper (22) einen Speicherkern (12), ein den Speicherkern (12) überfangendes Topsheet (13) und ein den Speicherkern unterfangendes Backsheet (14) aufweist.

13. Hygieneartikel nach einem der vorstehenden Ansprüche, **dadurch gekennzeichnet, dass** der jeweilige distale Rand (71) der Bündchenelemente (7) an dessen vorderen und/oder hinteren Endabschnitt (74) auf dem Topsheet (13) festgelegt ist.

14. Hygieneartikel nach einem der vorstehenden Ansprüche, **dadurch gekennzeichnet, dass** der jeweilige distale Rand (71) der Bündchenelemente (7) nach innen, in Richtung der zentralen Längsachse (L) des Artikels geneigt ist, in dem der jeweilige Endabschnitt (74) nach innen, in Richtung der Längsachse (L) des Artikels gelegt auf dem Topsheet (13) festgelegt ist.

15. Hygieneartikel nach einem der vorstehenden Ansprüche, **dadurch gekennzeichnet, dass** ein erster elastischer Beinöffnungsabschnitt (81) einer jeweilige Beinöffnung (8 a, b) im flachgelegten Zustand vor der herstellerseitigen Verbindung der Längsseitenrandabschnitte (16, 18) ausgehend von einem Längsseitenrand (12, 14) des Vorderteils (6) oder Rückenteils (4) des Artikels in einer Kurve entlang eines Umfangs (40) einer jeweiligen Beinöffnung (8 a, b) in Richtung auf eine Schrittmittellinie (42) vorgesehen ist.

16. Hygieneartikel nach einem der vorstehenden Ansprüche, **dadurch gekennzeichnet, dass** ein dritter elastischer Beinöffnungsabschnitt (83) einer jeweilige Beinöffnung (8 a, b) im flachgelegten Zustand vor der herstellerseitigen Verbindung der Längsseitenrandabschnitte (16, 18) ausgehend von einem Längsseitenrand (12, 14) des Vorderteils (6) oder Rückenteils (4) des Artikels in einer Kurve entlang eines Umfangs (40) einer jeweiligen Beinöffnung (8 a, b) in Richtung auf eine Schrittmittellinie (42) vorgesehen ist.

17. Hygieneartikel nach Anspruch 16, **dadurch gekennzeichnet, dass** an den ersten Beinöffnungsabschnitt (81) anschließend ein zweiter elastischer Beinöffnungsabschnitt (82) vorgesehen ist und daran anschließend der dritte elastische Beinöffnungsabschnitt (83) vorgesehen ist.

18. Hygieneartikel nach Anspruch 17, **dadurch gekennzeichnet, dass** der zweite elastische Beinöffnungsabschnitt (82) durch faden- oder bandförmige Elastifizierungsmittel (84) elastifiziert ist, die im Wesentlichen parallel zur zentralen Längsachse (L) angeordnet sind.

19. Hygieneartikel nach Anspruch 15 oder 16, **dadurch gekennzeichnet, dass** die jeweiligen Endbereiche des zweiten elastischen Beinöffnungsabschnittes (82) von den jeweiligen Endbereichen des ersten (81) und/oder dritten elastischen Beinöffnungsabschnittes (83) um eine Länge A beabstandet sind, wobei A vorzugsweise mindestens 10 mm, und weiter vorzugsweise mindestens 15 mm beträgt.

20. Hygieneartikel nach einem oder mehreren der vorstehenden Ansprüche, **dadurch gekennzeichnet, dass** die Elastifizierungsmittel (84) des zweiten elastischen Beinöffnungsabschnittes (82) am Topsheet (13) und/oder am Backsheet (14) des Absorptionskörpers (22) fixiert sind.

21. Hygieneartikel nach einem der vorstehenden Ansprüche, **dadurch gekennzeichnet, dass** die Elastifizierungsmittel (84) des zweiten elastischen Beinöffnungsabschnittes (82) in einer Falte des Backsheets (14) fixiert sind.

22. Hygieneartikel nach einem der Ansprüche 17-21, **dadurch gekennzeichnet, dass** die Längserstreckung des elastischen Bündchenabschnittes (9) um höchstens 8 cm, insbesondere um höchstens 6 cm, insbesondere um höchstens 4 cm, insbesondere um höchsten 2 cm größer ist als die Längserstreckung des Abstandes C zwischen dem ersten elastischen Beinöffnungsabschnitt (81) und dem dritten elastischen Beinöffnungsabschnitt (83).

## Claims

1. Disposable absorbent pant-type hygiene product (2) having a hip edge (20) which is continuously closed in the circumferential direction and forms a hip opening, and having leg openings (8a,b), and having a front part (6) and rear part (4) with longitudinal-side edge sections (16, 18), and having a crotch region (10) which is located between front part (6) and rear part (4); the hip edge (20), which is continuously closed in the circumferential direction, and the leg openings (8a,b) being formed in that longitudinal-side edge sections (16, 18) of a front part (6) and of a rear part (4) are connected to one another by the manufacturer, and having an absorbent body (22), a respective leg opening (8a,b) having an elastic leg-opening section (81); and cuff elements (7) being provided on both sides of the absorbent body (22) and made to extend in the longitudinal direction (28) of said absorbent body (22); a respective cuff element (7) having an elastic cuff section (9), and wherein
a) a respective cuff element (7) has at least one non-elastic cuff section (11) and, after being put on the body of the user, the elastic leg-opening section (81) crosses the respective non-elastic cuff section (11), or
b) the longitudinal extent of the respective cuff element (7) is dimensioned such that, after being put on the body of the user, a crossing of the elastic leg-opening section (81) with the respective cuff element (7) does not take place.

2. Hygiene product according to Claim 1, **characterized in that** a respective cuff element (7) has two non-elastic cuff sections (11), a first non-elastic cuff section being closer to the front hip edge (20a) and a second non-elastic cuff section being closer to the rear hip edge (20b) than the elastic cuff section (9).

3. Hygiene product according to Claim 1 or 2, **characterized in that** a respective cuff element (7) has a single elastic cuff section (9) which is at least in portions positioned in the crotch region (10).

4. Hygiene product according to one of the preceding claims, **characterized in that** a respective cuff element (7) has at least two elastic cuff sections, the elastic cuff sections being spaced apart from one another by non-elastic cuff sections.

5. Hygiene product according to one of the preceding claims, **characterized in that** the elastic cuff section (9) and/or the elastic leg-opening section (81) is or are elasticated by thread-like or tape-like elastication means (73, 86).

6. Hygiene product according to one of the preceding claims, **characterized in that** the non-elastic cuff section (11) is obtained by elastication means previously made to extend there having been severed.

7. Hygienic article according to Claim 6, **characterized in that** the severing is achieved by a multiplicity of cuts.

8. Hygiene product according to one or more of the preceding claims, **characterized in that** the non-elastic cuff section (11) is obtained by elastication means previously made to extend there having been relieved of their elasticating effect by application of heat and/or pressure and/or laser influence and/or ultrasound.

9. Hygiene product according to one of the preceding claims, **characterized in that** the non-elastic cuff section (11) is obtained by elastication means which extend there being disposed without tension.

10. Hygiene product according to one of the preceding claims, **characterized in that** the non-elastic cuff section (11) is obtained by no elastication means being disposed there at least in portions.

11. Hygiene product according to one of the preceding claims, **characterized in that** the cuff elements (7) have a distal edge (71) and a proximal edge (72) and the elastic cuff section is elasticated by the thread-like or tape-like elastication means (73) disposed on the distal edge.

12. Hygiene product according to one of the preceding claims, **characterized in that** the absorbent body (22) has a storage core (12), a top sheet (13), which runs over the storage core (12), and a back sheet (14), which runs under the storage core (12).

13. Hygiene product according to one of the preceding claims, **characterized in that** the respective distal edge (71) of the cuff elements (7) is fixed at its front and/or rear end section (74) on the top sheet (13).

14. Hygiene product according to one of the preceding claims, **characterized in that** the respective distal edge (71) of the cuff elements (7) is inclined, in the direction of the central longitudinal axis (L) of the article in which the respective end section (74) laid inwards, in the direction of the longitudinal axis (L) of the article is fixed on the top sheet (13).

15. Hygiene product according to one of the preceding claims, **characterized in that** a first elastic leg-opening section (81) of a respective leg opening (8a,b) is provided in the laid-flat state before the connection of the longitudinal-side edge sections (16, 18) by the manufacturer, proceeding from a longitudinal-side edge (12, 14) of the front part (6) or rear part (4) of the article in a curve along a circumference (40) of a respective leg opening (8a,b) in the direction of a crotch centre line (42).

16. Hygiene product according to one of the preceding claims, **characterized in that** a third elastic leg-opening section (83) of a respective leg opening (8a,b) is provided in the laid-flat state before the connection of the longitudinal-side edge sections (16, 18) by the manufacturer, proceeding from a longitudinal-side edge (12, 14) of the front part (6) or rear part (4) of the article in a curve along a circumference (40) of a respective leg opening (8a,b) in the direction of a crotch centre line (42).

17. Hygiene product according to Claim 16, **characterized in that** adjacent to the first leg-opening section (81) a second elastic leg-opening section (82) is provided, and adjacent to said second elastic leg-opening section (82) the third elastic leg-opening section (83) is provided.

18. Hygiene product according to Claim 17, **characterized in that** the second elastic leg-opening section (82) is elasticated by thread-like or tape-like elastication means (84) which are located substantially parallel to the central longitudinal axis (L).

19. Hygiene product according to either Claim 15 or 16, **characterized in that** the respective end regions of the second elastic leg-opening section (82) are spaced at a length A from the respective end regions of the first elastic leg-opening section (81) and/or third elastic leg-opening section (83), A being to preferably at least 10 mm, and more preferably at least 15 mm.

20. Hygiene product according to one or more of the preceding claims, **characterized in that** the elastication means (84) of the second elastic leg-opening section (82) are fixed on the top sheet (13) and/or on the back sheet (14) of the absorbent body (22).

21. Hygiene product according to one or more of the preceding claims, **characterized in that** the elastication means (84) of the second leg-opening section (82) are fixed in a fold of the back sheet (14).

22. Hygiene product according to one of Claims 17-21, **characterized in that** the longitudinal extent of the elastic cuff section (9) is greater by at most 8 cm, in particular by at most 6 cm, in particular by at most 4 cm, in particular by at most 2 cm than the longitudinal extent of the distance C between the first elastic leg-opening section (81) and the third elastic leg-opening section (83).

## Revendications

1. Article hygiénique (2), absorbant, jetable, en forme de culotte, présentant
un bord de hanche (20) formant une ouverture de hanche et fermé de manière continue dans la direction périphérique, des ouvertures de jambe (8a, b), une partie avant (6) et une partie arrière (4) présentant des parties (16, 18) de bord latéral longitudinal, une zone d'entrejambe (10) disposée entre la partie avant (6) et la partie arrière (4),
le bord de hanche (20) fermé de manière continue dans la direction périphérique et les ouvertures de jambe (8a, b) étant formées en reliant l'une à l'autre chez le fabricant les parties (16, 18) de bord latéral longitudinal d'une partie avant (6) et d'une partie arrière (4), et
un corps absorbant (22),
chaque ouverture de jambe (8a, b) présentant une partie élastique (81) d'ouverture de jambe et des éléments de collerette (7) étant prévus des deux côtés du corps absorbant (22) et s'étendant dans le sens (28) de sa longueur, chaque élément de collerette (7) présentant une partie élastique (9) de collerette,
a) chaque élément de collerette (7) présentant au moins une partie (11) non élastique de collerette et, après placement sur le corps de l'utilisateur, la partie élastique (81) d'ouverture de jambe croisant la partie non élastique de collerette (11) ou
b) l'extension longitudinale de chaque élément de collerette (7) est dimensionnée de telle sorte qu'après placement sur le corps de l'utilisateur, la partie élastique (81) d'ouverture de jambe ne croise pas l'élément de collerette (7) concerné.

2. Article hygiénique selon la revendication 1, **caractérisé en ce que** chaque élément de collerette (7) présente deux parties non élastiques (11) de collerette, une première partie non élastique de collerette étant disposée plus près du bord de hanche (20a) voisin et une deuxième partie non élastique de collerette étant disposée plus près du bord de hanche arrière (20b) que la partie élastique de collerette (9).

3. Article hygiénique selon les revendications 1 ou 2, **caractérisé en ce que** chaque élément de collerette (7) présente une seule partie élastique (9) de collerette partiellement disposée au niveau de l'entrejambe (10).

4. Article hygiénique selon l'une des revendications précédentes, **caractérisé en ce que** chaque élément de collerette (7) présente au moins deux parties élastiques de collerette, les parties élastiques de collerette étant maintenues à distance mutuelle par des parties non élastiques de collerette.

5. Article hygiénique selon l'une des revendications précédentes, **caractérisé en ce que** la partie élastique (9) de collerette et/ou la partie élastique (81) d'ouverture de jambe sont élastifiées par des moyens d'élastification (73, 86) en forme de fil ou de ruban.

6. Article hygiénique selon l'une des revendications précédentes, **caractérisé en ce que** la partie non élastique (11) de collerette est obtenue en y séparant des moyens d'élastification préalablement étirés.

7. Article hygiénique selon la revendication 6, **caractérisé en ce que** la séparation est obtenue par plusieurs découpes.

8. Article hygiénique selon l'une ou plusieurs des revendications précédentes, **caractérisé en ce que** la partie non élastique (11) de collerette est obtenue en retirant à des moyens d'élastification préalablement étendus leur effet d'élastification par application de chaleur, d'une pression, d'un laser ou d'ultrasons.

9. Article hygiénique selon l'une des revendications précédentes, **caractérisé en ce que** la partie non élastique (11) de collerette est obtenue en n'exerçant pas de tension sur les moyens d'élastification qui s'y étendent.

10. Article hygiénique selon l'une des revendications précédentes, **caractérisé en ce que** la partie non élastique (11) de collerette est obtenue en n'y disposant pas de moyen d'élastification, au moins localement.

11. Article hygiénique selon l'une des revendications précédentes, **caractérisé en ce que** les éléments de collerette (7) présentent un bord distal (71) et un bord proximal (72) et **en ce que** la partie élastique de collerette est élastifiée par les moyens d'élastification (73) en forme de fil ou de ruban disposés sur le bord distal.

12. Article hygiénique selon l'une des revendications précédentes, **caractérisé en ce que** le corps absorbant (22) présente une âme d'accumulation (12), une feuille supérieure (13) qui recouvre l'âme d'accumulation (12) et une feuille de dos (14) qui recouvre par le bas l'âme d'accumulation.

13. Article hygiénique selon l'une des revendications précédentes, **caractérisé en ce que** chaque bord distal (71) des éléments de collerette (7) est fixé sur la feuille supérieure (13) par sa partie d'extrémité avant et/ou sa partie d'extrémité arrière (74).

14. Article hygiénique selon l'une des revendications précédentes, **caractérisé en ce que** chaque bord distal (71) des éléments de collerette (7) est incliné vers l'intérieur en direction de l'axe central longitudinal (L) de l'article par le fait que la partie d'extrémité (74) concernée est fixée sur la feuille supérieure (13) en étant placée vers l'intérieur en direction de l'axe longitudinal (L) de l'article.

15. Article hygiénique selon l'une des revendications précédentes, **caractérisé en ce qu'**une première partie élastique (81) de chaque ouverture de jambe (8a, b) est prévue en position aplatie avant la liaison chez le fabricant entre les parties (16, 18) de bord latéral longitudinal, partant d'un bord latéral longitudinal (12, 14) de la partie avant (6) ou de la partie arrière (4) de l'article, suivant une courbe qui longe la périphérie (40) de l'ouverture de jambe (8a, b) concernée en direction d'une ligne centrale (42) d'entrejambe.

16. Article hygiénique selon l'une des revendications précédentes, **caractérisé en ce qu'**une troisième partie élastique (83) de chaque ouverture de jambe (8a, b) est prévue en position aplatie avant la liaison chez le fabricant entre les parties (16, 18) de bord latéral longitudinal, partant d'un bord latéral longitudinal (12, 14) de la partie avant (6) ou de la partie arrière (4) de l'article, suivant une courbe qui longe la périphérie (40) de l'ouverture de jambe (8a, b) concernée en direction d'une ligne centrale (42) d'entrejambe.

17. Article hygiénique selon la revendication 16, **caractérisé en ce qu'**une deuxième partie élastique (82) d'ouverture de jambe est prévue sur la première partie (81) d'ouverture de jambe et **en ce que** la troisième partie élastique (83) d'ouverture de jambe y est ensuite prévue.

18. Article hygiénique selon la revendication 17, **caractérisé en ce que** la deuxième partie élastique (82) d'ouverture de jambe est élastifiée par des moyens d'élastification (84) en forme de fil ou de ruban disposés essentiellement en parallèle à l'axe longitudinal central (L).

19. Article hygiénique selon les revendications 15 ou 16, **caractérisé en ce que** chaque partie d'extrémité de la deuxième partie élastique (82) d'ouverture de jambe est maintenue à une distance A des parties d'extrémité respectives de la première partie (81) d'ouverture de jambe et/ou de la troisième partie élastique (83) d'ouverture de jambe, A représentant de préférence au moins 10 mm et de manière encore plus préférable au moins 15 mm.

20. Article hygiénique selon l'une ou plusieurs des revendications précédentes, **caractérisé en ce que** les moyens d'élastification (84) de la deuxième partie élastique (82) d'ouverture de jambe sont fixés sur la feuille supérieure (13) et/ou sur la feuille arrière (14) du corps absorbant (22).

21. Article hygiénique selon l'une des revendications précédentes, **caractérisé en ce que** les moyens d'élastification (84) de la deuxième partie élastique (82) d'ouverture de jambe sont fixés dans un pli de la feuille arrière (14).

22. Article hygiénique selon l'une des revendications 17 à 21, **caractérisé en ce que** l'extension longitudinale de la partie élastique (9) de collerette est d'au plus 8 cm, notamment d'au plus 6 cm, en particulier d'au plus 4 cm et notamment d'au plus 2 cm plus grande que l'extension longitudinale de la distance C entre la première partie élastique (81) d'ouverture de jambe et la troisième partie élastique (83) d'ouverture de jambe.
